# DEMANDE DE BREVET EUROPEEN

(11) **EP 1 254 935 A2**
(43) Date de publication de la demande: **06.11.2002**
(21) Numéro de dépôt: 02291086.3
(22) Date de dépôt: 30.04.2002
(51) Int. Cl.: C09H 3/00, C08L 89/06, C09H 9/02, A61K 9/48

(54) **Gélatines à bon pouvoir glissant, procédés pour leur préparation et leurs applications**

(30) Priorité: 02.05.2001 FR 0105862
(71) Demandeur: Degussa Health & Nutrition Holding France SAS, 92100 Boulogne-Billancourt (FR)
(72) Inventeur: Lafargue, Olivier, 84800 Isle-sur-Sorgue (FR); David, Jacky, 84800 Isle-sur-Sorgue (FR); Takerkart, Georges, 92130 Issy-les-Moulineaux (FR)
(74) Mandataire: Gillard, Marie-Louise

(57) **Abrégé**

L'invention concerne des gélatines à pouvoir glissant amélioré, des procédés pour leur préparation et leurs applications notamment dans la fabrication de capsules.

Lesdites gélatines contiennent des composés tensioactifs anioniques ou non ioniques.

## Description

La présente invention concerne des gélatines à pouvoir glissant amélioré, des procédés pour leur préparation et leurs applications notamment dans la fabrication de capsules.

La préparation des gélules à partir de gélatine a été décrite par le français A.B. MOTHES dans le brevet n° 9690 déposé en 1834. Le procédé s'est ensuite répandu et de nombreux brevets ont été déposés.

Dans les années qui ont suivi, les procédés de préparation de gélules à partir de gélatine ont été constamment améliorés pour satisfaire des spécifications techniques de plus en plus exigeantes.

La gélatine est le principal constituant de la capsule. Il s'agit de gélatines de haute pureté préparées à partir de matières premières collagéniques situées dans la peau et les os des animaux. Il existe deux types de gélatine : celle de type A, de point isoélectrique compris entre 7,5 et 9, extraite par traitement avec une solution aqueuse chaude d'acide, généralement H₂SO₄ de couennes de porc, de peaux bovines ou d'os concassés, dépoussiérés et déminéralisés; celle de type B, de point isoélectrique voisin de 5 obtenue par traitement des os déminéralisés ou de peaux bovines par une solution aqueuse basique, chaux ou soude généralement.

La capsule dure se compose de deux parties cylindriques, à savoir un corps avec un fond hémisphérique et un chapeau de la même forme mais plus courte.

Avant le remplissage, les corps des capsules passent par un goulot pour s'insérer dans un logement positionné sur un disque qui tourne pour permettre le remplissage. A ce stade, il est important d'éviter le ralentissement dans les goulots ainsi que les interactions de type adhérence ou phénomènes électrostatiques des corps des capsules avec la poudre.

Le diamètre interne du chapeau étant pratiquement égal au diamètre externe du corps, quand les deux parties sont rassemblées, on obtient un réservoir de volume très reproductible. Etant donné qu'il s'agit d'un ajustement serré et que l'assemblage se fait sur des machines à débit élevé, il est crucial que les surfaces devant se recouvrir glissent parfaitement l'une par rapport à l'autre. Si cela n'est pas le cas, les deux parties de la capsule sont rejetées, ce qui réduit la cadence de la machine et augmente le taux de rejet.

Le problème à résoudre consiste donc à améliorer la propriété de glisse des parois de la capsule vis-à-vis des matériaux des machines (goulots), des poudres et des autres capsules.

La propriété de glisse des parois de la capsule peut être évaluée en préparant des films de la gélatine utilisée.

Le test consiste à mesurer l'inclinaison à partir de laquelle deux films de la même gélatine glissent l'un contre l'autre. Des techniques plus élaborées peuvent également être utilisées pour caractériser l'état de surface telle que la technique dite AFM pour « Atomic Force Microscopy » ou microscopie à force atomique telle que décrite par exemple dans S. Magonov et al., Surface Analysis with STM and AFM, VCH Ed., 1996.

Le test de glisse mis au point par les inventeurs est le suivant :

On pèse dans un flacon 30 g de gélatine, puis 70 g d'eau déminéralisée sont ajoutés. Après homogénéisation, la solution est laissée au repos (pour gonflement ) pendant 30 min. Les flacons sont alors placés dans un bain à 65°C. Après 2 h, la solution est agitée puis incubée 19 h dans un bain marie à 50°C.

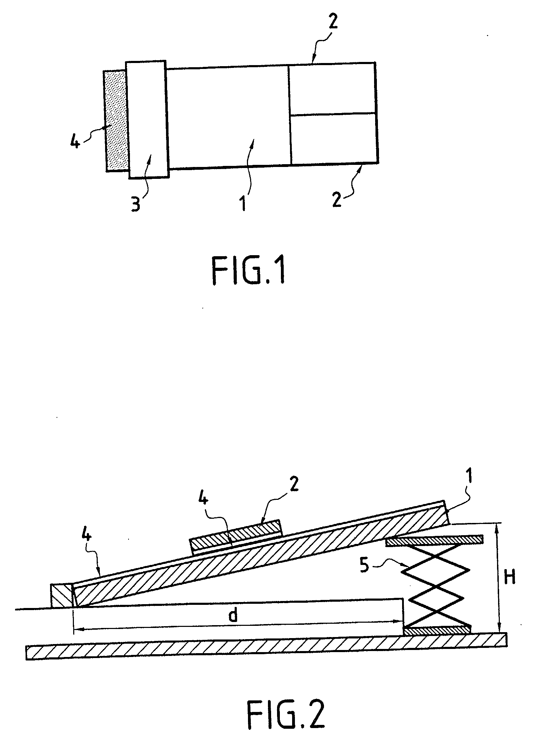

Trois films de gélatine sont ensuite préparés à l'aide d'un applicateur de films représenté sur la figure 1.

Celle-ci est une vue de dessus montrant :
- en (1), une plaque de verre de 200 mm x 200 mm,
- en (2), 2 plaques de verre de 100 mm x 50 mm,
- en (3), un dévidoir dont la fente est réglée à 0,8 mm, et
- en (4), le film de gélatine.

La solution de gélatine est versée dans le dévidoir qui se déplace à la vitesse de 5 cm/s.

Les 3 plaques, une fois séparées, sont placées dans une étuve à 27°C, sous une atmosphère à 40 % d'humidité relative pendant 18 h.

Pour mesurer les propriétés glissantes, on utilise le système représenté sur la figure 2.

La plaque (1) recouverte de film de gélatine (4) est disposée de manière à réaliser un plan incliné, le film de gélatine étant sur la face supérieure et on fait glisser sur celle-ci la plaque (2) également recouverte d'un film de gélatine selon une inclinaison réglée par un système élévateur (5).

La distance d est de 130 mm.

La hauteur est ensuite diminuée progressivement jusqu'à ce que la petite plaque ne glisse plus spontanément. On détermine alors une valeur Hₘₘ caractéristique des propriétés glissantes, les propriétés de glisse étant d'autant meilleures que cette valeur Hₘₘ sera faible.

On a pu mettre en évidence par ce test que certaines gélatines avaient des propriétés de glisse remarquables alors que d'autres ne glissaient pas du tout, et une échelle de classement a pu être établie.

Il a été ainsi trouvé que la propriété de glisse pouvait être reliée à la présence de certains composés dans la gélatine. Leur nature a pu être déterminée : il s'agit de composés tensioactifs anioniques naturels issus de la matière première utilisée, mais qui ne sont présents dans la gélatine qu'en très faible quantité. De manière surprenante, on a trouvé qu'il était possible d'augmenter significativement la teneur en composés tensioactifs anioniques dans la gélatine par rapport à la teneur naturelle. Une méthode de dosage a été mise au point. Elle consiste à les complexer avec un ammonium quaternaire (type chlorure de cétyl diméthyl ammonium benzyle) et à doser l'excès d'ammonium par le bleu de bromophénol en UV à 605 nm.

L'invention concerne donc, selon un premier aspect, une gélatine à pouvoir glissant amélioré contenant une teneur augmentée en composés tensioactifs anioniques naturels, lesdits composés améliorant l'état de surface des films préparés à partir de cette gélatine.

La teneur en composés tensioactifs anioniques naturels d'une gélatine classique est généralement inférieure à environ 50 ppm.

Avantageusement, la quantité de composés tensioactifs anioniques de la gélatine à pouvoir glissant amélioré selon l'invention est comprise entre 200 et 10 000 ppm, de préférence entre 300 et 4 000 ppm.

Lesdits composés tensioactifs anioniques sont notamment des dérivés d'acides gras tels que par exemple d'acides stéarique, palmitique, oléique, myristique ou pentadécanoïque.

De manière surprenante, il a également été trouvé que ces composés tensioactifs anioniques étaient libérés au cours de certaines étapes de la fabrication dans les gélatines et que leur teneur pouvait être augmentée par certains traitements à l'aide d'enzymes protéolytiques. On obtient ainsi des gélatines selon l'invention ayant des propriétés de glisse remarquables et conduisant à des performances accrues lors de la fabrication des capsules.

L'invention concerne donc, selon un aspect ultérieur, un procédé de préparation d'une gélatine telle que définie plus haut, dans lequel les composés tensioactifs anioniques sont libérés in situ par traitement enzymatique d'une matière première collagénique pendant l'extraction à chaud de la gélatine.

Le traitement consiste à ajouter dans la cuve d'extraction une faible quantité d'au moins une enzyme protéolytique et à contrôler la température et la durée d'extraction pour limiter la dégradation de la gélatine et préserver en particulier la viscosité souhaitée.

A titre d'exemples de protéases, on peut citer non limitativement les protéases neutres, alcalines ou acides, ou une composition enzymatique en contenant, telles que celles disponibles dans le commerce, comme par exemple la neutrase ® fournie par NOVO qui comprend principalement une métalloprotéase de *Bacillus subtilis*, l'alcalase ® fournie par NOVO renfermant de la subtilisine A, ou encore la pronase ou la papaïne.

De préférence, le pH lors de cette addition est de 5 à 8, en particulier 7 et la température est égale ou supérieure à environ 50°C, de préférence de 60 à 80°C.

La durée de l'extraction sera de préférence de 2 à 6 h.

Selon un aspect de l'invention, le traitement enzymatique peut être effectué à un stade avancé de l'extraction, une ou plusieurs étapes d'extraction ayant été préalablement réalisées sans présence d'enzyme.

Avantageusement, ledit traitement enzymatique est effectué après extraction d'au moins 30 à 40% de la gélatine totale.

Aux fins de l'invention, la gélatine est extraite de matières premières collagéniques par des procédés classiques tels que décrits, par exemple dans « The Science and Technology of Gelatin », A.G. Ward et A. Courts, Academic Press Ed., 1977.

De préférence, ladite matière première collagénique n'est pas une matière première telle quelle mais a, préalablement à l'extraction, subi un traitement de préparation acide ou alcaline .

Par « préparation acide » ou « préparation alcaline », on entend les traitements préalables à l'extraction décrits dans des procédés classiques tel que par exemple le trempage de la matière première pendant une durée plus ou moins prolongée dans un bain d'acide minéral ou organique, ou dans une solution alcaline telle qu'une solution d'hydroxyde de sodium ou de calcium.

On peut également se référer à cet égard à l'ouvrage « The Science and Technology of Gelatin » cité plus haut.

Des matières premières collagéniques avantageusement utilisables sont par exemple l'osséine (os déminéralisé), la peau bovine, la couenne porcine ou la peau de poisson, l'osséine chaulée étant préférée.

Selon un aspect préféré, la gélatine est extraite d'osséine chaulée et le traitement enzymatique est effectué in situ en cours d'extraction en utilisant de 0,2 g à 5 g d'enzyme(s)/tonne d'osséine chaulée.

L'invention concerne également, selon un de ses aspects, l'utilisation de la gélatine à pouvoir glissant amélioré telle que définie précédemment pour la fabrication de films et de capsules.

Selon un autre de ses aspects, l'invention concerne également un procédé de préparation de la gélatine telle que définie plus haut, consistant à ajouter des composés tensioactifs anioniques solubles en milieu aqueux à une solution de gélatine après extraction. L'utilisation de composés tensioactifs anioniques solubles en présence de gélatine présente l'avantage de préserver la limpidité de la solution de gélatine.

De préférence, lesdits composés tensioactifs anioniques sont ajoutés à raison de 200 à 10 000 ppm, de préférence 300 à 4 000 ppm.

De préférence, lesdits composés tensioactifs anioniques sont choisis parmi les sels d'acides gras tels que le stéarate, le palmitate, l'oléate, le myristate ou le pentadécanoate de sodium ou de potassium, les sels de sodium étant préférés, ou encore les phospholipides tels que les lécithines.

Selon un aspect ultérieur, l'invention concerne également une gélatine à pouvoir glissant amélioré contenant des composés tensioactifs non ioniques.

En effet, on a également trouvé que l'ajout de composés tensioactifs non ioniques à une solution de gélatine après extraction permettait d'améliorer le pouvoir glissant de ladite gélatine et d'obtenir gélatine pour laquelle la valeur Hₘₘ des films réalisés avec cette gélatine était significativement diminuée par rapport à la gélatine non supplémentée en composés tensioactifs non ioniques.

De préférence, on ajoute de 200 à 10 000 ppm, notamment de 300 à 4 000 ppm desdits composés tensioactifs non ioniques à la solution de gélatine.

L'invention a donc également pour objet un procédé d'obtention d'une telle gélatine tel que décrit ci-dessus, ainsi que l'utilisation de cette gélatine pour la fabrication de films et de capsules.

De préférence, les composés tensioactifs non ioniques sont choisis parmi les dérivés de sorbitan tels que par exemple le monostéarate de sorbitan (Span® 60) ou le monooléate de sorbitan (Span® 80).

L'invention est illustrée par les exemples ci-après :

### Exemple 1:

15 tonnes d'osséine chaulée, obtenues selon un procédé classique, sont chargées avec de l'eau dans une cuve d'extraction. 10 g de métalloprotéase neutre de *B. subtilis* (B500 de GIST-BROCADES) sont ajoutés dans la cuve dès la montée à température à 75°C, le pH étant maintenu à 6,5. Après extraction de la gélatine durant 3 à 4 h, la solution est pasteurisée afin d'inactiver l'enzyme, filtrée, déminéralisée puis gélifiée et séchée de manière classique dans un séchoir ventilé à température contrôlée.

On obtient ainsi une gélatine ayant une teneur en composés tensioactifs anioniques de 827 ppm et une valeur Hₘₘ = 25 mm, ce qui correspond à un test de glisse satisfaisant.

### Exemple 2 :

400 ppm de palmitate de sodium sont ajoutés à une solution à 30 % de gélatine de couenne de porc ne présentant pas de propriétés glissantes.

On obtient ainsi une gélatine ayant une teneur en composés tensioactifs anioniques de 700 ppm et une valeur Hₘₘ = 30 mm, alors qu'elle était de 70 mm avant ajout.

### Exemple 3:

1000 ppm de polysorbate 80 (SIGMA) sont ajoutés à une solution à 30% de gélatine extraite d'osséine chaulée ne présentant pas de propriétés glissantes. On obtient ainsi une gélatine ayant une valeur Hₘₘ = 30 mm, alors qu'elle était de 70 mm avant ajout.

## Revendications

1. Gélatine à pouvoir glissant amélioré, **caractérisée en ce qu'**elle contient une teneur augmentée en composés tensioactifs anioniques naturels, lesdits composés améliorant l'état de surface des films préparés à partir de ladite gélatine.

2. Gélatine selon la revendication 1, **caractérisée en ce que** la teneur en composés tensioactifs anioniques est comprise entre 200 et 10000 ppm, de préférence entre 300 et 4000 ppm.

3. Procédé de préparation d'une gélatine selon l'une des revendications 1 ou 2, **caractérisé en ce que** les composés tensioactifs anioniques sont libérés in situ par traitement enzymatique d'une matière première collagénique pendant l'extraction à chaud de la gélatine.

4. Procédé de préparation d'une gélatine selon l'une des revendications 1 à 3, **caractérisé en ce que** ladite matière première collagénique a, préalablement à l'extraction, subi un traitement de préparation acide ou alcaline .

5. Procédé selon la revendication 4, **caractérisé en ce que** ladite matière première collagénique est choisie parmi l'osséine, la peau bovine, la couenne porcine et la peau de poisson.

6. Procédé selon les revendications 3, 4 ou 5, **caractérisé en ce que** la (les) dite(s) enzyme(s) utilisée(s) pour le traitement enzymatique est (sont) choisie(s) parmi les protéases neutres alcalines ou acides telles que les métalloprotéases de *B. subtilis,* la subtilisine A, la pronase, la papaïne et les compositions en contenant.

7. Procédé selon l'une quelconque des revendications 3 à 6, **caractérisée en ce que** la (les)dite(s) enzyme(s) est (sont) ajoutée(s) dans la cuve d'extraction à un pH de 5 à 8 et à une température égale ou supérieure à 50°C.

8. Procédé selon l'une quelconque des revendications 3 à 7, **caractérisé en ce que** le traitement enzymatique est effectué à un stade avancé de l'extraction, une ou plusieurs étapes d'extraction ayant été préalablement réalisées sans présence d'enzyme.

9. Procédé selon la revendication 8, **caractérisé en ce que** le traitement enzymatique est effectué après extraction d'au moins 30 à 40% de la gélatine totale.

10. Procédé selon l'une quelconque des revendications 3 à 9, **caractérisé en ce que** la gélatine est extraite d'osséine chaulée et **en ce que** le traitement enzymatique est effectué in situ en cours d'extraction en utilisant de 0,2 g à 5 g d'enzyme(s) par tonne d'osséine chaulée.

11. Procédé de préparation d'une gélatine selon l'une quelconque des revendications 1 ou 2, **caractérisé en ce que** l'on ajoute à une solution de gélatine, après extraction, des composés tensioactifs anioniques solubles en milieu aqueux.

12. Procédé selon la revendication 11, **caractérisé en ce que** lesdits composés tensioactifs anioniques sont ajoutés à raison de 200 à 10 000 ppm, de préférence 300 à 4 000 ppm.

13. Procédé selon les revendications 11 ou 12, **caractérisé en ce que** lesdits composés tensioactifs anioniques sont choisis parmi les sels d'acides gras et les phospholipides.

14. Gélatine à pouvoir glissant amélioré, **caractérisée en ce qu'**elle contient des composés tensioactifs non ioniques qui améliorent l'état de surface des films préparés à partir de ladite gélatine.

15. Gélatine selon la revendication 14, **caractérisée en ce que** la teneur en composés tensioactifs non ioniques est comprise entre 200 et 10 000 ppm, de préférence entre 300 et 4 000 ppm.

16. Procédé d'obtention d'une gélatine selon les revendications 14 ou 15, **caractérisé en ce qu'**on ajoute à une solution de gélatine, après extraction, des composés tensioactifs non ioniques afin d'améliorer le pouvoir glissant de ladite gélatine.

17. Procédé selon la revendication 16, **caractérisé en ce que** lesdits composés tensioactifs non ioniques sont ajoutés à raison de 200 à 10 000 ppm, de préférence de 300 à 4 000 ppm.

18. Procédé selon les revendications 16 ou 17, **caractérisé en ce que** lesdits composés tensioactifs non ioniques sont choisis parmi les dérivés de sorbitan, tels que le monostéarate de sorbitan et le monooléate de sorbitan.

19. Utilisation d'une gélatine selon l'une des revendications 1, 2, 14 ou 15 pour la fabrication de films.

20. Utilisation d'une gélatine selon l'une des revendications 1, 2, 14 ou 15 pour la fabrication de capsules.
